# EUROPEAN PATENT APPLICATION

(11) **EP 4 591 923 A1**
(43) Date of publication of application: **30.07.2025**
(21) Application number: 22959550.9
(22) Date of filing: 22.09.2022
(51) Int. Cl.: A61N 1/365

(54) **WIRELESS STIMULUS CONTROL DEVICE, STENT, WIRELESS STIMULUS CONTROL SYSTEM, WIRELESS STIMULUS CONTROL METHOD, AND WIRELESS STIMULUS CONTROL PROGRAM**

(71) Applicant: HICKY, Inc., Tokyo 113-0033 (JP)
(72) Inventor: HAYASHI, Kentaro, Tokyo 113-0033 (JP); IEKI, Hirotaka, Tokyo 113-0033 (JP); KONDO, Yusuke, Tokyo 113-0033 (JP); INAGAKI, Daisuke, Tokyo 113-0033 (JP)
(74) Representative: Hoffmann Eitle
(86) International application number: PCT/JP2022/035309
(87) International publication number: WO 2024/062586

(57) **Abstract**

Provided is a wireless stimulation control device, including: a living body analysis unit for generating, on the basis of living body information regarding a living body acquired from a sensor, living body analysis information indicating a result of analyzing the state of the living body; a stimulation control unit for generating, on the basis of the living body analysis information, stimulation control information regarding control of stimulation of a nearby nerve of an in vivo stent, with the stimulation using an induced current and being performed by the stent that is configured to generate the induced current on the basis of an electric field or magnetic field; and a wireless power supply unit for supplying, on the basis of at least one of the living body analysis information and the stimulation control information, an electric field or magnetic field to the stent so as to generate the induced current.

## Description

### Technical Field

The present disclosure relates to a wireless stimulation control device, a stent, a wireless stimulation control system, a wireless stimulation control method, and a wireless stimulation control program.

### Background Art

In the prior art, a system is known, which controls a device that has been implanted in a body by means of wireless power supply, which uses an electric field or magnetic field from outside a living body, to the device implanted in the body.

For example, the device described in Patent Literature 1 supplies electric power to a medical device main body through a power supply coil having a cylindrical coil and an annular ring surrounding an outer circumference of the cylindrical coil, with this power supply coil being configured to wirelessly supply power to a power reception coil provided in the medical device main body, which is implanted in a living body, from outside the living body through electromagnetic induction.

### Citation List

### Patent Literature

Patent Literature 1: Patent Publication JP-A-2017-164192

### Summary

However, in the device described in Patent Literature 1, the power supply to the medical device main body implanted in the body is based on the operation of an operator, so control according to the state of the living body is not taken into consideration, which becomes a burden on both the operator and the patient.

Therefore, an object of the present disclosure is to provide a wireless stimulation control device, a stent, a wireless stimulation control system, a wireless stimulation control method, and a wireless stimulation control program that can perform control according to the state of a living body.

A wireless stimulation control device according to one aspect of the present disclosure includes: a living body analysis unit for generating, on the basis of living body information regarding a living body acquired from a sensor, living body analysis information indicating a result of analyzing the state of the living body; a stimulation control unit for generating, on the basis of the living body analysis information, stimulation control information regarding control of stimulation of a nearby nerve of an in vivo stent, with the stimulation using an induced current and being performed by the stent that is configured to generate the induced current on the basis of an electric field or magnetic field; and a wireless power supply unit for supplying, on the basis of at least one of the living body analysis information and the stimulation control information, an electric field or magnetic field to the stent so as to generate the induced current.

A wireless stimulation control method according to one aspect of the present disclosure includes, with a computer: generating, on the basis of living body information regarding a living body acquired from a sensor, living body analysis information indicating a result of analyzing a state of the living body; generating, on the basis of the living body analysis information, stimulation control information regarding control of stimulation of a nearby nerve of an in vivo stent, with the stimulation using an induced current and beings performed by the stent that is configured to generate the induced current on the basis of an electric field or magnetic field; and supplying, on the basis of at least any of the living body analysis information and the stimulation control information, an electric field or magnetic field to the stent so as to generate the induced current.

A wireless stimulation control program according to one aspect of the present disclosure causes a computer to implement: a living body analysis unit for generating, on the basis of living body information regarding a living body acquired from a sensor, living body analysis information indicating a result of analyzing a state of the living body; a stimulation control unit for generating, on the basis of the living body analysis information, stimulation control information regarding control of stimulation of a nearby nerve of an in vivo stent, with the stimulation using an induced current and being performed by the stent that is configured to generate an induced current on the basis of an electric field or magnetic field; and a wireless power supply unit for supplying, on the basis of at least one of the living body analysis information and the stimulation control information, an electric field or magnetic field to the stent so as to generate the induced current.

Note that as used in the present disclosure, the term "unit" not only signifies physical means but also includes cases where functions of the "unit" are implemented by software. In addition, functions of one "unit" or device may be implemented by two or more physical means, devices or pieces of software, and moreover functions of two or more "units" or devices may be implemented by one physical means, one device, or one piece of software.

### Disclosure

According to the present disclosure, it is possible to provide a wireless stimulation control device, a wireless stimulation control method, and a wireless stimulation control program that can perform control according to the state of a living body.

### Brief Description of Drawings

[Figure 1] Figure 1 is a diagram showing an overview of processing in a wireless stimulation control system 100 according to an embodiment of the present disclosure.
[Figure 2] Figure 2 is a diagram showing the configuration of the wireless stimulation control system 100 according to an embodiment of the present disclosure.
[Figure 3] Figure 3 is a diagram showing an example of living body information stored in a storage unit 210.
[Figure 4] Figure 4 is a diagram showing an example of living body analysis information stored in the storage unit 210.
[Figure 5] Figure 5 is a diagram showing an example of stimulation control information stored in the storage unit 210.
[Figure 6] Figure 6 is a diagram showing an example of stimulation of a nearby nerve performed by a stent 300.
[Figure 7] Figure 7 is a sequence chart showing an example of processing in the wireless stimulation control system 100.
[Figure 8] Figure 8 is a sequence chart showing an example of processing in the wireless stimulation control system 100.

### Description of Embodiments

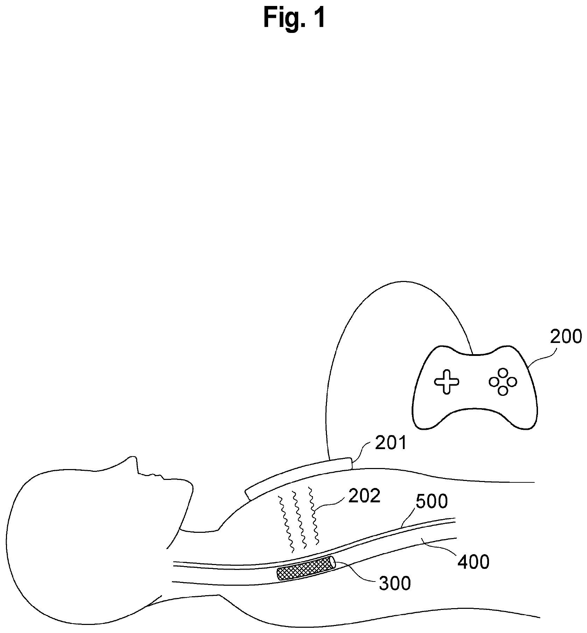

Preferred embodiments of the present disclosure will be described with reference to the accompanying drawings. Figure 1 is a diagram showing an overview of processing in a wireless stimulation control system 100 according to an embodiment of the present disclosure.

The wireless stimulation control system 100 is an information processing system implemented by a wireless stimulation control program, and includes: a wireless stimulation control device 200 for controlling, using an electric field or magnetic field, stimulation performed by an in vivo stent 300; and the in vivo stent 300 for stimulating a nearby nerve in response to the electric field or magnetic field.

The wireless stimulation control device 200 includes, for example, a pad 201 attached to a living body. First, the wireless stimulation control device 200 acquires living body information regarding a living body from a sensor (for example, a sensor included in the pad 201). The wireless stimulation control device 200 analyzes the living body information and supplies to the in vivo stent 300 an electric field or magnetic field 202through the pad 201, so as to generate an induced current. That is, in this case, the pad 201 has the function of a sensor, and acquires living body information, and a function of supplying an electric field or magnetic field. Note that the sensor function and the function of supplying an electric field or magnetic field may be implemented by the same means (for example, the pad 201), or may be implemented by separate means.

The stent 300 is introduced into the body (for example, into a blood vessel 400). Near the stent 300, there is a nerve 500.

The stent 300 generates an induced current on the basis of the electric field or magnetic field 202, and performs stimulation of the nerve 500 by using the induced current.

The wireless stimulation control system 100 can be applied, for example, to patients with sleep apnea syndrome.

Specifically, the wireless stimulation control device 200 acquires respiration information regarding the respiration of a patient from a sensor, for example. The wireless stimulation control device 200 analyzes the living body information, and supplies an electric field or magnetic field 202 to the stent 300, which is placed in a blood vessel 400 (for example, a phrenic vein) near a nerve 500 (for example, the phrenic nerve) of the patient, through a pad 201, so as to generate an induced current. At this time, the wireless stimulation control device 200 supplies the electric field or magnetic field 202 to the stent 300, for example, when the patient's breathing has stopped or is unstable.

The stent 300 then generates an induced current on the basis of the electric field or magnetic field 202, and the stent 300 performs stimulation on the nerve 500 by using the induced current. This causes the patient's diaphragm to move and the patient's breathing resumes or stabilizes.

Note that the wireless stimulation control system can also be applied to individuals other than patients with sleep apnea syndrome.

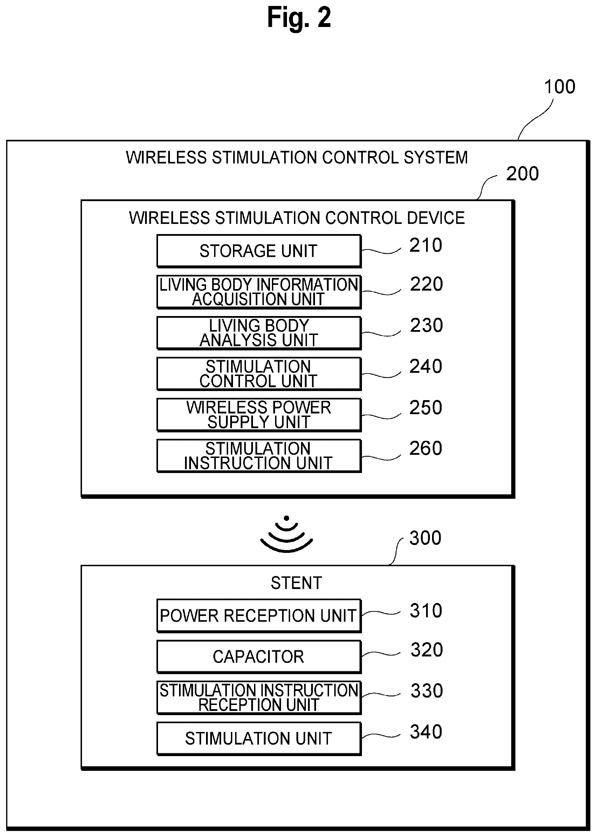

Figure 2 is a diagram showing the configuration of the wireless stimulation control system 100 according to an embodiment of the present disclosure. The wireless stimulation control system 100 includes the wireless stimulation control device 200 and the stent 300.

The wireless stimulation control device 200 is an information processing device that analyzes living body information and supplies an electric field or magnetic field to the stent 300, so as to generate an induced current.

**The** wireless stimulation control device 200 includes a storage unit 210, a living body information acquisition unit 220, a living body analysis unit 230, a stimulation control unit 240, a wireless power supply unit 250 and a stimulation instruction unit 260. Each unit of the wireless stimulation control device 200 can be implemented, for example, by using a storage area or by a processor executing a program stored in the storage area.

**The** stent 300 is a stent placed in the body and configured to generate an induced current on the basis of an electric field or a magnetic field. **The** stent 300 stimulates, in response to the electric field or magnetic field supplied from the wireless stimulation control device 200, a nearby nerve by using an induced current.

Here, the stent 300 includes, for example, a cylindrical structure made of metal. **The** stent 300 can, for example, expand a tubular portion (for example, a lumen such as a blood vessel) in the body from inside by means of the cylindrical structure. Note that the stent 300 may be expanded by a balloon or may expand without a balloon (so-called self-expansion) by pressing against the vessel wall, for example.

**In** addition, the stent 300 includes a conductor portion that allows a current to flow and an insulator portion that does not allow a current to flow.

For example, the stent 300 may have conductors at both ends and an insulator at a central part. Specifically, electrodes attached to both ends of the stent 300 may function as conductors.

**The** stent 300 includes a power reception unit 310, a capacitor 320, a stimulation instruction reception unit 330, and a stimulation unit 340.

Note that the stent 300 in the present embodiment may consist solely of a stent main body (for example, a cylindrical structure main body made of metal) or may include a stent main body (for example, a cylindrical structure main body made of metal) and certain additional parts. **That** is, functions of each functional unit (for example, the power reception unit 310, the capacitor 320, the stimulation instruction reception unit 330, and the stimulation unit 340) of the stent 300 may be implemented by the stent main body or by additional parts attached to the stent main body. **In the** present embodiment, the stent main body, as well as the stent main body and the additional parts as a whole, is simply referred to as the stent.

Note that although only one stent 300 is shown in Figure 2, there may be multiple stents 300 in the body. Moreover, the living body in which the stent 300 is placed is not limited, and may, for example, be a human body. **In** addition, the position where the stent 300 is placed is not limited, and may, for example, be within a blood vessel.

Next, details of processing in the wireless stimulation control system 100 will be described. First, details of processing in the wireless stimulation control device 200 will be described.

**The** storage unit 210 stores information processed in the wireless stimulation control device 200. **The** storage unit 210 can store, for example, living body information, living body analysis information and stimulation control information, which will be described below.

**The** living body information acquisition unit 220 acquires living body information regarding a living body from a sensor and stores same in the storage unit 210.

**The** living body information acquisition unit 220 may acquire the living body information from, for example, a sensor attached to the chest of a living body or a sensor disposed near a living body (for example, the back of the living body or near the pillow). **In** addition, the living body information acquisition unit 220 may also acquire the living body information from, for example, a sensor provided in a space where a living body is located (for example, in a bedroom of the living body).

**The** living body information acquisition unit 220 may acquire respiration information regarding the respiration of the living body and take same as the living body information, for example.

**The** living body information includes, for example, time information and sensor information. **The** time information is, for example, information indicating the time when the living body information acquisition unit 220 acquires the living body information from a sensor or the time when a sensor acquires the living body information.

**The** sensor information is information regarding a living body that is acquired by a sensor. **The** sensor information may, for example, be acceleration information regarding an acceleration indicating the movement of a living body. **That** is, when a sensor is attached to the chest of a living body, the sensor information may be acceleration information indicating the up-and-down movement of the chest. Therefore, the sensor information may detect whether a living body undergoes respiration.

Note that the sensor information may be any information regarding a living body that a sensor may acquire, such as the body temperature, pulse wave, or blood pressure of the living body, or the concentration of specific substances (for example, oxygen or carbon dioxide in blood or exhaled air) in the living body. **In** addition, the sensor information may, for example, be information based on electromagnetic waves (for example, millimeter-wave radar) transmitted and received by a sensor.

In addition, the sensor function of a sensor may be implemented by the sensor included in the pad 201 attached to the living body, for example, or it may be implemented by other means. The sensor may be located inside or outside the living body. In addition, the sensor may also be included in the wireless stimulation control device 200. Moreover, the sensor function, and a wireless power supply function of the wireless power supply unit 250, which will be described below, may be implemented by the same means (for example, the pad 201), or may be implemented by separate means.

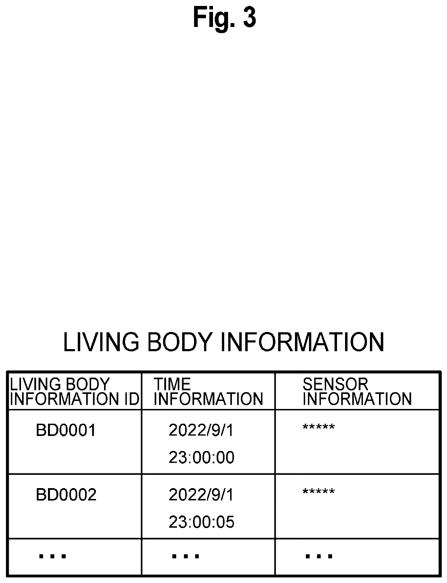

Figure 3 is a diagram showing an example of the living body information stored in the storage unit 210. The living body information stored in the storage unit 210 includes, for example, living body information ID, time information, sensor information, and the like. The living body information ID is information for identifying the living body information stored in the storage unit 210.

The living body analysis unit 230 generates, on the basis of the living body information, living body analysis information indicating the result of analyzing the state of the living body, and stores same in the storage unit 210.

Here, the state of the living body is the state of the living body corresponding to the living body information. The state of the living body may be, for example, the respiration state corresponding to the respiration information. The respiration state may be, for example, a state based on whether respiration occurs, the intensity of respiration, or the stability of respiration, and the like.

That is, the living body analysis unit 230 can generate, on the basis of the respiration information, living body analysis information indicating the result of analyzing the respiration state of the living body, for example.

The living body analysis information may be information corresponding to each piece of living body information or information corresponding to multiple pieces of living body information. That is, the living body analysis information may be information acquired by analyzing each piece of living body information in accordance with each piece of time information shown in the living body information, or information acquired by analyzing multiple pieces of living body information within a certain period.

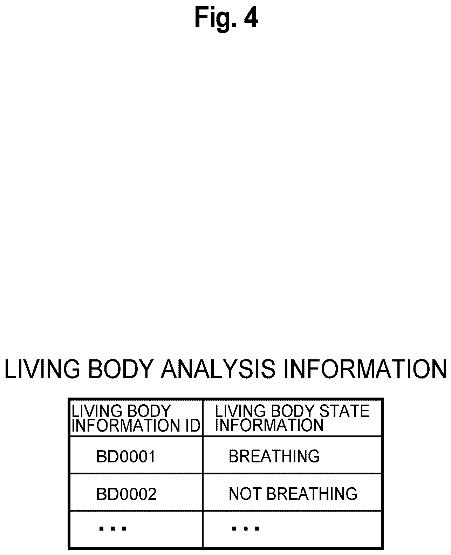

Figure 4 is a diagram showing an example of the living body analysis information stored in the storage unit 210. **The** living body analysis information stored in the storage unit 210 includes, for example, living body information **ID** and living body state information.

The living body state information is information regarding the state of the living body. In the case where the living body state information indicates the respiration state of the living body, the living body state information may be "breathing" or "not breathing" indicating whether respiration occurs, for example, or it may be information indicating the intensity or stability of respiration.

Note that in Figure 4, the living body analysis information is shown as information corresponding to each piece of living body information at each time point, but the living body analysis information may also be information corresponding to multiple pieces of living body information within a certain period.

**The** stimulation control unit 240 generates, on the basis of the living body analysis information, stimulation control information regarding the control of stimulation of a nearby nerve of the stent 300, which stimulation uses an induced current and is performed by the stent 300, and stores same in the storage unit 210.

Here, the stimulation control information is information regarding the control of stimulation of a nearby nerve, which stimulation is performed by the stent 300, such as the appropriateness of the stimulation, the timing of the stimulation, and details of the stimulation, which stimulation is controlled by the wireless stimulation control device 200. **That** is, the stent 300 performs stimulation on the basis of the content indicated in the stimulation control information, according to the stimulation control information.

**The** stimulation control unit 240 can generate, on the basis of the living body analysis information, stimulation control information such that stimulation performed by the stent 300 is not carried out, for example, when it is determined that stimulation is not needed (for example, when the living body is breathing normally). **In** such case, the stimulation control unit 240 may also choose not to generate stimulation control information.

**In** addition, the stimulation control unit 240 can generate, on the basis of the living body analysis information, stimulation control information for stimulation performed by the stent 300, for example, when it is determined that stimulation is needed (for example, when the living body's breathing has stopped). **In** such case, the stimulation control unit 240 may control, on the basis of living body analysis information, the content of the stimulation performed by the stent 300.

Specifically, patterns (for example, respiration patterns) of the state of the living body may be analyzed on the basis of the living body analysis information, and the stimulation control information for stimulation performed by the stent 300 at an appropriate timing, for an appropriate duration, at an appropriate intensity, and at an appropriate waveform may be generated. **That** is, the stimulation control information may include information regarding at least any one of the timing, duration, intensity, and waveform of the stimulation performed by the stent 300.

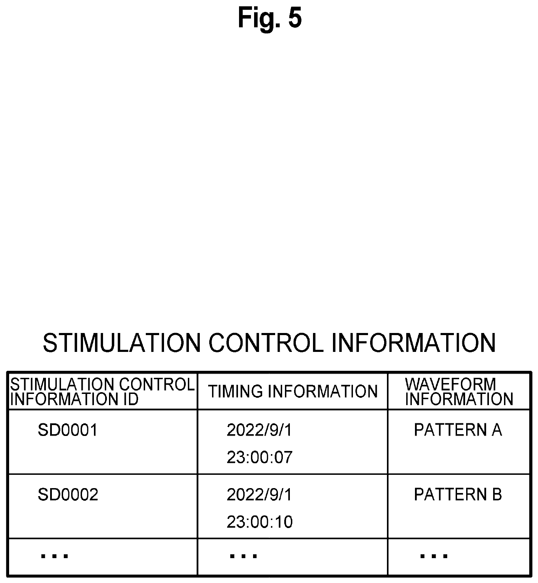

Figure 5 is a diagram showing an example of the stimulation control information stored in the storage unit 210. **The** stimulation control information stored in the storage unit 210 includes, for example, stimulation control information ID, timing information and waveform information.

The stimulation control information ID is information for identifying the stimulation control information stored in the storage unit 210. The timing information is information indicating the timing for performing stimulation by the stent 300, such as information indicating the time when stimulation is performed. The waveform information is information indicating the waveform (for example, stimulation intensity, stimulation time) of the stimulation performed by the stent 300, and may be information (for example, "Pattern A" or "Pattern B") indicating any one of a plurality of waveform patterns that are set in advance.

Note that the timing information may be information indicating conditions based on the living body analysis information. That is, the timing information may also be information indicating that stimulation should be performed when conditions based on the living body analysis information (for example, conditions related to the depth of breathing) are satisfied (for example, when breathing becomes shallow).

In addition, the waveform may be determined by factors such as the intensity or frequency of the stimulation. That is, the stimulation unit 340 of the stent 300, which will be described below, can stimulate a nearby nerve by using an induced current that has the intensity or frequency corresponding to the waveform indicated in the waveform information.

The wireless power supply unit 250 supplies, on the basis of at least one of the living body analysis information and the stimulation control information, an electric field or magnetic field to the stent 300 so as to generate an induced current. That is, the wireless power supply unit 250 performs wireless power supply on the stent 300 on the basis of at least one of the living body analysis information and the stimulation control information.

The wireless power supply unit 250 supplies an electric field or magnetic field to the stent 300, thus providing electric power to the stent 300 for stimulation performed by the stent 300. The wireless power supply unit 250 supplies an electric field or magnetic field to the stent 300, and can perform wireless power supply utilizing methods such as electromagnetic induction, magnetic resonance, electric field coupling, or radio wave reception.

Here, the wireless power supply unit 250 may supply an electric field or magnetic field to the stent 300 through the pad 201 attached to the living body, or it may supply an electric field or magnetic field to the stent 300 by other means. Note that the sensor function of a sensor, and a wireless power supply function of the wireless power supply unit 250 may be implemented by the same means (for example, the pad 201), or may be implemented by separate means.

**In** addition, the wireless power supply unit 250 can supply an electric field or magnetic field to the stent 300 on the basis of the living body analysis information.

Specifically, the wireless power supply unit 250 may supply an electric field or magnetic field to the stent 300, on the basis of the living body analysis information, when it is determined that stimulation is needed (for example, when the living body's breathing has stopped).

**In** addition, the wireless power supply unit 250 may supply an electric field or magnetic field to the stent 300 on the basis of the stimulation control information.

Specifically, the wireless power supply unit 250 can supply, on the basis of the stimulation control information, an electric field or magnetic field to the stent 300 for stimulation performed by the stent 300 at a timing indicated in the stimulation control information (particularly, the timing information), for example.

**In** addition, the wireless power supply unit 250 can supply an electric field or magnetic field to the stent 300 on the basis of the living body analysis information and the stimulation control information.

Specifically, the wireless power supply unit 250 may supply, on the basis of the living body analysis information, an electric field or magnetic field to the stent 300 for stimulation performed by the stent 300 at a timing indicated in the stimulation control information (particularly, the timing information), when it is determined that stimulation is needed (for example, when the living body's breathing has stopped), for example.

**In** this way, the wireless power supply unit 250 can supply an electric field or a magnetic field to the stent 300, thus providing electric power for stimulation performed by the stent 300. Therefore, the wireless stimulation control device 200 and the stent 300 do not need to be connected by leads, such that the movement of the living body (such as a patient) into which the stent 300 is introduced is not limited by leads.

**The** stimulation instruction unit 260 supplies, on the basis of the stimulation control information, an electric field or magnetic field to the stent 300 so as to transmit a stimulation instruction signal that instructs the stimulation of a nearby nerve of the stent 300.

Here, the stimulation instruction signal is a signal transmitted from the wireless stimulation control device 200 to the stent 300 by means of an electric field or magnetic field (for example, electromagnetic induction). **The** stent 300 can perform stimulation of a nearby nerve on the basis of the stimulation instruction signal.

Note that the wireless stimulation control device 200 may not include the stimulation instruction unit 260. **In** such case, the wireless stimulation control device 200 may replace the wireless power supply by means of the wireless power supply unit 250 with the stimulation instruction signal. **That** is, the stimulation unit 340 of the stent 300, which will be described below, can stimulate a nearby nerve of the stent 300 in response to the wireless power supply by means of the wireless power supply unit 250 (that is, for example, in response to the receiving of power by the power reception unit 310 of the stent 300, which will be described below).

Next, details of processing in the stent 300 will be described.

**The** power reception unit 310 receives power in response to an electric field or magnetic field for generating an induced current used for the stimulation of a nearby nerve of the stent 300. The power reception unit 310 receives power in response to the electric field or magnetic field supplied by the wireless power supply unit 250 of the wireless stimulation control device 200, for example.

The power reception unit 310 is, for example, a metal coil, and can receive electric power in response to an electric field or magnetic field through electromagnetic induction. For example, the power reception unit 310 may be attached to the inner side of the cylindrical structure included in the stent 300, or may be attached to other parts of the stent 300.

The capacitor 320 stores electricity in response to the receiving of power by the power reception unit 310. The capacitor 320 may be implemented by components referred to, for example, as capacitors, condensers, or batteries. The capacitor 320 may be attached, for example, to the inner side of the cylindrical structure included in the stent 300.

Note that the stent 300 may not include the capacitor 320. In such case, as described below, the stent 300 may perform stimulation by means of the stimulation unit 340 in response to the receiving of power by the power reception unit 310 without storing electricity in response to the receiving of power by the power reception unit 310.

The stimulation instruction reception unit 330 receives a stimulation instruction signal that instructs stimulation of the nearby nerve and is transmitted using an electric field or magnetic field. The stimulation instruction reception unit 330 can receive a stimulation instruction signal transmitted by means of an electric field or magnetic field supplied by the stimulation instruction unit 260 of the wireless stimulation control device 200, for example.

The stimulation instruction reception unit 330 may be implemented by an IC (Integrated Circuit) chip included in the stent 300, for example. The IC chip may be attached, for example, to the inner side of the cylindrical structure included in the stent 300.

The stimulation unit 340 stimulates, in response to the receiving of power by the power reception unit 310, a nearby nerve of the stent 300 by using an induced current generated on the basis of an electric field or magnetic field.

Specifically, the stimulation unit 340 causes a current to flow, for example, from one conductor portion (for example, an electrode) in the stent 300 to the other conductor portion in response to the receiving of power by the power reception unit 310. At this time, for example, one of the conductor portions is connected to the power reception unit 310 via a lead. Therefore, in response to the receiving of power by the power reception unit 310, a current flows to this conductor portion through the lead. Then, the current flows to the other conductor portion via the nearby nerve of the stent 300.

More specifically, for example, if both ends of the stent 300 are conductors and the central part is an insulator, and a current flows from the conductor portion at one end of the stent 300 to the conductor portion at the other end thereof, the current cannot flow through the insulator portion at the central part, so the current flows via a nearby nerve. As a result, the nearby nerve is stimulated.

Moreover, a current may also flow between the conductor portions of each of the multiple stents 300. When a current flows through a conductor portion of one stent and a conductor portion of another stent, the current flows via the nearby nerve. As a result, the nearby nerve is stimulated.

The nearby nerve stimulated by the stimulation unit 340 is not limited, but may be, for example, the phrenic nerve, hypoglossal nerve, vagus nerve, or sacral nerve.

The stimulation unit 340 can perform stimulation in response to the receiving of power by the power reception unit 310 without storing the electric power received by the power reception unit 310. That is, in this case, the power received by the power reception unit 310 acts as a switch for the stimulation performed by the stimulation unit 340.

In addition, the stimulation unit 340 can stimulate a nearby nerve in response to a stimulation instruction signal. That is, when the stimulation instruction reception unit 330 receives a stimulation instruction signal, the stimulation unit 340 releases, in response to the stimulation instruction signal, the electric power stored in the capacitor 320. As a result, the nearby nerve can be stimulated.

Also, the stimulation unit 340 can stimulate the nearby nerve when the amount of electricity stored in the capacitor 320 reaches a predetermined threshold value, which is a predetermined threshold value greater than the amount of power received per unit of time by the power reception unit 310.

That is, the power reception unit 310 receives, per unit of time, an amount of power smaller than the amount of power received and required for stimulation performed by the stimulation unit 340, and stores same in the capacitor 320. Then, when the amount of stored electricity reaches a predetermined threshold value, the stimulation unit 340 stimulates the nearby nerve. As a result, the stimulation performed by the stimulation unit 340 can be performed while reducing the amount of power received per unit of time by the power reception unit 310 (that is, for example, the amount of power supplied per unit of time by the wireless power supply unit 250 of the wireless stimulation control device 200).

Note that a stent 300 may be assembled outside the living body with each functional unit first and then introduced into the living body, or a stent 300 including some functional units may be introduced into the living body first, and then other functional units may be assembled in the living body using a specified technique.

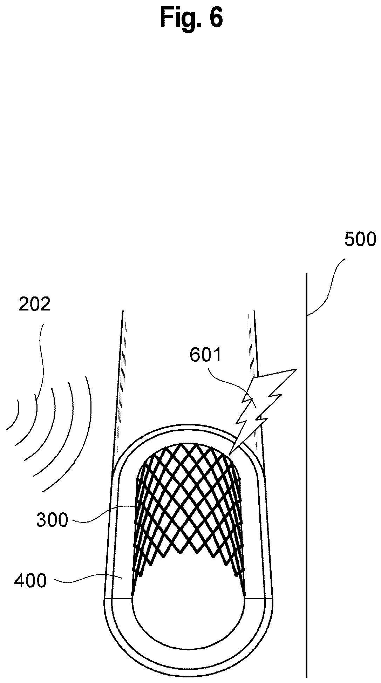

Figure 6 is a diagram showing an example of stimulation of a nearby nerve performed by a stent 300. Figure 6 schematically shows the positional relationship among the stent 300, the blood vessel 400 and the nerve 500.

**The** stent 300 is a cylindrical piece of metal and is located within the blood vessel 400, for example. **In** response to the power received by the power reception unit 310 by using the electric field or magnetic field 202, a current 601 flows through the conductor portion of the stent 300. When the current passes via the nearby nerve 500, the nearby nerve is stimulated.

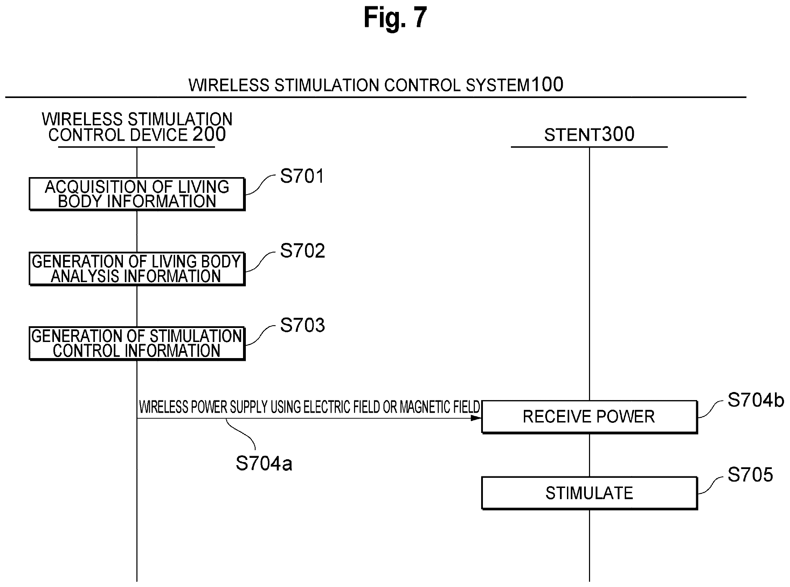

Figure 7 is a sequence chart showing an example of processing in the wireless stimulation control system 100. Figure 7 is a sequence chart showing an example of the processing when the stimulation performed by the stent 300 is carried out in response to the wireless power supply by means of the wireless stimulation control device 200.

First, the living body information acquisition unit 220 of the wireless stimulation control device 200 acquires living body information from a sensor (S701). The living body analysis unit 230 generates living body analysis information on the basis of the living body information (S702). The stimulation control unit 240 generates stimulation control information on the basis of the living body analysis information (S703). **The** wireless power supply unit 250 supplies, on the basis of at least one of the living body analysis information and the stimulation control information, an electric field or magnetic field to the stent 300 so as to generate an induced current (S704a).

Next, the power reception unit 310 of the stent 300 receives power in response to the electric field or magnetic field supplied from the wireless power supply unit 250 of the wireless stimulation control device (S704b). **The** stimulation unit 340 of the stent 300 stimulates, in response to the receiving of power by the power reception unit 310, a nearby nerve by using an induced current generated on the basis of the electric field or magnetic field (S705).

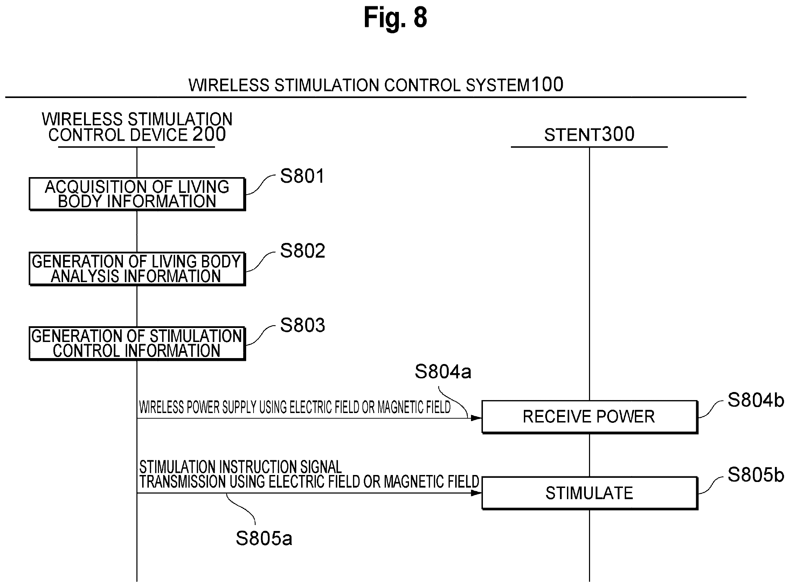

Figure 8 is a sequence chart showing an example of processing in the wireless stimulation control system 100. Figure 8 is a sequence chart showing an example of the processing when the stimulation performed by the stent 300 is carried out in response to the stimulation instruction signal from the wireless stimulation control device 200.

First, the living body information acquisition unit 220 of the wireless stimulation control device 200 acquires living body information from a sensor (S801). **The** living body analysis unit 230 generates living body analysis information on the basis of the living body information (S802). **The** stimulation control unit 240 generates stimulation control information on the basis of the living body analysis information (S803).

Subsequently, the wireless power supply unit 250 supplies, on the basis of at least one of the living body analysis information and the stimulation control information, an electric field or magnetic field to the stent 300 so as to generate an induced current (S804a). The power reception unit 310 of the stent 300 receives power in response to the electric field or magnetic field supplied from the wireless power supply unit 250 of the wireless stimulation control device (S804b). At this time, the received electric power may be stored in the capacitor 320 of the stent 300.

Then, the stimulation instruction unit 260 of the wireless stimulation control device 200 supplies, on the basis of the stimulation control information, an electric field or magnetic field to the stent 300 so as to transmit a stimulation instruction signal that instructs the stimulation of a nearby nerve of the stent 300 (S805a). The stimulation instruction reception unit 330 of the stent 300 receives the stimulation instruction signal, and the stimulation unit 340 stimulates the nearby nerve (S805b).

Next, an application example of the wireless stimulation control system 100 will be described.

The wireless stimulation control system 100 can be applied, for example, to the treatment of patients with sleep apnea syndrome.

Specifically, the living body information acquisition unit 220 of the wireless stimulation control device 200 acquires respiration information regarding the respiration of a patient from a sensor. Then, the living body analysis unit 230 analyzes the respiration state of the patient on the basis of the respiration information, and generates living body analysis information.

The stent 300 is, for example, introduced into a blood vessel near the phrenic nerve of the patient. Note that the stent 300 may also be introduced into a blood vessel near other nerves (for example, the vagus nerve) involved in the respiration of a living body. The stimulation unit 340 of the stent 300 stimulates a nearby nerve (for example, the phrenic nerve) according to the processing of the wireless stimulation control device 200. As a result, the normal respiration of the patient is encouraged.

In the treatment of patients with sleep apnea syndrome, a method is known in which a stent including leads is introduced into the body, and then power supply and stimulation control are performed on the stent from an external device via the leads. However, in this method, since a stent including leads is introduced into the body, the movement of the patient is restricted, and the burden on the patient increases. The wireless stimulation control system 100 controls the stent 300 through wireless power supply by means of the wireless stimulation control device 200, eliminating the need for leads, so that the burden on the patient can be reduced. In addition, the wireless stimulation control system 100 controls the stent 300 on the basis of living body analysis information, thereby reducing the operational burden on the operator.

Moreover, compared to positive pressure therapy used for the treatment of patients with sleep apnea syndrome, there is less burden on patients. In positive pressure therapy, the patient needs to wear a mask over his/her mouth, which increases the burden on him/her, especially during sleep. The wireless stimulation control system 100 treats sleep apnea syndrome through wireless control by using an electric field or magnetic field on the stent 300 introduced into the body, thereby reducing the burden on the patient.

That is, an embodiment of the present disclosure is a wireless stimulation control method for the treatment of patients with sleep apnea syndrome by using a wireless stimulation control device 200, wherein the wireless stimulation control device 200 generates, on the basis of respiration information regarding the respiration of a patient, which is acquired from a sensor and taken as living body information, living body analysis information indicating the result of analyzing the respiration state of the patient; generates, on the basis of the living body analysis information, stimulation control information regarding the control of stimulation of a nearby nerve of an in vivo stent 300, which stimulation uses an induced current and is performed by the stent 300, which is configured to generate the induced current on the basis of an electric field or magnetic field; and supplies, on the basis of at least one of the living body analysis information and the stimulation control information, an electric field or magnetic field to the stent 300 so as to generate the induced current.

**In** addition, an embodiment of the present disclosure is a wireless stimulation control method for the treatment of patients with sleep apnea syndrome by using a wireless stimulation control device 200 and a stent 300, wherein on the basis of respiration information regarding the respiration of a patient, which is acquired from a sensor and taken as living body information, living body analysis information indicating the result of analyzing the respiration state of the patient is generated; on the basis of the living body analysis information, stimulation control information regarding the control of stimulation of a nearby nerve of an in vivo stent 300, which stimulation uses an induced current and is performed by the stent 300, which is configured to generate the induced current on the basis of an electric field or magnetic field, is generated; and on the basis of at least one of the living body analysis information and the stimulation control information, an electric field or magnetic field is supplied to the stent 300 so as to generate the induced current, and wherein the stent 300 introduced into the body receives power in response to the electric field or magnetic field, and stimulates the nearby nerve by using the induced current generated on the basis of the electric field or magnetic field in response to the receiving of the power.

Note that the wireless stimulation control system 100 may also be applied to the treatment of other diseases.

An embodiment of the present disclosure has been explained above. The wireless stimulation control device 200 can supply, on the basis of at least one of the living body analysis information and the stimulation control information, an electric field or magnetic field to the stent 300 so as to generate an induced current, wherein the living body analysis information is generated on the basis of the living body information acquired from a sensor, and the stimulation control information regarding the control of stimulation performed by the stent 300 is generated on the basis of the living body analysis information. Therefore, the wireless stimulation control device 200 can perform control according to the state of the living body.

In addition, the wireless stimulation control device 200 can supply, to the stent 300, an electric field or magnetic field for transmitting a stimulation instruction signal that instructs the stimulation of the nearby nerve. As a result, stimulation instructions can be given to the stent 300 independently of the presence or absence of wireless power supply.

The wireless stimulation control device 200 can generate the stimulation control information that includes information regarding at least any one of the timing, duration, intensity, and waveform of the stimulation performed by the stent 300. Thus, the wireless stimulation control device 200 can control the details of the stimulation of the nearby nerve performed by the stent 300.

In addition, the wireless stimulation control device 200 can generate, on the basis of the respiration information, which is acquired from a sensor and taken as the living body information, the living body analysis information by analyzing the respiration state of patients with sleep apnea syndrome, and can control the stimulation of the phrenic nerve performed by the stent 300. This allows for the control of treatment of patients with sleep apnea syndrome according to the state of the living body.

In addition, the stent 300 can receive power in response to an electric field or magnetic field for generating an induced current, and stimulate a nearby nerve by using the induced current. Thus, the in vivo stent 300 can stimulate the nearby nerve without leads.

In addition, the stent 300 can stimulate the nearby nerve on the basis of the stimulation instruction signal transmitted using an electric field or magnetic field. As a result, the stent 300 can stimulate the nearby nerve on the basis of the control of the wireless stimulation control device 200.

In addition, the wireless stimulation control system 100 includes the wireless stimulation control device 200 and the stent 300 according to an embodiment of the present disclosure. Therefore, the stimulation performed by the in vivo stent 300 can be controlled according to the state of the living body.

Note that the present embodiment is intended to facilitate the understanding of the present disclosure but not to limit the interpretation of the present disclosure. The present disclosure can be modified or improved without departing from the gist thereof, and the present disclosure also includes equivalents thereof.

### Reference Signs List

- 100: Wireless stimulation control system
- 200: Wireless stimulation control device
- 210: Storage unit
- 220: Living body information acquisition unit
- 230: Living body analysis unit
- 240: Stimulation control unit
- 250: Wireless power supply unit
- 260: Stimulation instruction unit
- 300: Stent
- 310: Power reception unit
- 320: Capacitor
- 330: Stimulation instruction reception unit
- 340: Stimulation unit

## Claims

1. A wireless stimulation control device, comprising:
a living body analysis unit for generating, on the basis of living body information regarding a living body acquired from a sensor, living body analysis information indicating a result of analyzing the state of the living body;
a stimulation control unit for generating, on the basis of the living body analysis information, stimulation control information regarding control of stimulation of a nearby nerve of an in vivo stent, with the stimulation using an induced current and being performed by the stent that is configured to generate the induced current on the basis of an electric field or magnetic field; and
a wireless power supply unit for supplying, on the basis of at least one of the living body analysis information and the stimulation control information, an electric field or magnetic field to the stent so as to generate the induced current.

2. **The** wireless stimulation control device according to claim 1, further comprising:
a stimulation instruction unit for supplying, on the basis of the stimulation control information, an electric field or magnetic field to the stent so as to transmit a stimulation instruction signal that instructs the stimulation of the nerve.

3. **The** wireless stimulation control device according to claim 1 or 2, wherein
the stimulation control information includes information regarding at least any of timing, duration, intensity, and waveform of the stimulation performed by the stent.

4. **The** wireless stimulation control device according to any one of claims 1 to 3, wherein
the living body analysis unit generates, on the basis of respiration information regarding respiration of a patient with sleep apnea syndrome, which is acquired from a sensor and taken as living body information, living body analysis information indicating a result of analyzing a respiration state of the patient; and
the nearby nerve is at least any of a phrenic nerve and a hypoglossal nerve.

5. A stent, which is introduced into the body, comprising:
a power reception unit for receiving power in response to an electric field or magnetic field for generating an induced current used for stimulating a nearby nerve of the stent; and
a stimulation unit for stimulating, in response to the receiving of power by the power reception unit, the nerve by using the induced current generated on the basis of the electric field or magnetic field.

6. **The** stent according to claim 5, further comprising:
a stimulation instruction reception unit for receiving a stimulation instruction signal that instructs stimulation of the nerve and that is transmitted using an electric field or magnetic field, wherein
the stimulation unit stimulates the nerve on the basis of the stimulation instruction signal.

7. **The** stent according to claim 5 or 6, further comprising:
a capacitor for storing electricity in response to the receiving of power by the power reception unit, wherein
the stimulation unit stimulates the nerve when an amount of the electricity stored in the capacitor reaches a predetermined threshold value, which is a predetermined threshold value greater than an amount of power received per unit of time by the power reception unit.

8. A wireless stimulation control system, comprising:
the wireless stimulation control device according to any one of claims 1 to 4; and
the stent according to any one of claims 5 to 7.

9. A wireless stimulation control method comprising, with a computer:
generating, on the basis of living body information regarding a living body acquired from a sensor, living body analysis information indicating a result of analyzing a state of the living body;
generating, on the basis of the living body analysis information, stimulation control information regarding control of stimulation of a nearby nerve of an in vivo stent, with the stimulation using an induced current and being performed by the stent that is configured to generate the induced current on the basis of an electric field or magnetic field; and
supplying, on the basis of at least any of the living body analysis information and the stimulation control information, an electric field or magnetic field to the stent so as to generate the induced current.

10. A wireless stimulation control program, causing a computer to implement:
a living body analysis unit for generating, on the basis of living body information regarding a living body acquired from a sensor, living body analysis information indicating a result of analyzing a state of the living body;
a stimulation control unit for generating, on the basis of the living body analysis information, stimulation control information regarding control of stimulation of a nearby nerve of an in vivo stent, with the stimulation using an induced current and being performed by the stent that is configured to generate an induced current on the basis of an electric field or magnetic field; and
a wireless power supply unit for supplying, on the basis of at least any of the living body analysis information and the stimulation control information, an electric field or magnetic field to the stent so as to generate the induced current.
